# EUROPEAN PATENT SPECIFICATION

(11) **EP 3 417 847 B1**
(45) Date of publication and mention of the grant of the patent: **12.10.2022**
(21) Application number: 18178575.9
(22) Date of filing: 19.06.2018
(51) Int. Cl.: A61P 31/02, A61K 9/00, A61P 27/02

(54) **DISINFECTANT COMPOSITION FOR OPHTHALMIC USE**
DESINFEKTIONSMITTEL ZUR VERWENDUNG FÜR DIE AUGEN
COMPOSITION DE DÉSINFECTANT À USAGE OPHTALMIQUE

(30) Priority: 20.06.2017 IT 201700068170
(43) Date of publication of application: 26.12.2018
(73) Proprietor: Iromed Group S.r.l., 00144 Roma (RM) (IT)
(72) Inventor: CARUSO, Ciro, 80124 Napoli NA (IT)
(74) Representative: de Benedetti, Jacopo

(56) References cited:
- US-A- 5 126 127
- US-A1- 2005 196 370
- KRAMER A ET AL: "EFFICACY AND TOLERANCE OF SELECTED ANTISEPTIC SUBSTANCES IN RESPECT OF SUITABILITY FOR USE ON THE EYE", DEVELOPMENTS IN OPHTHALMOLOGY, KARGER, BASEL, CH, vol. 33, 1 January 2002 (2002-01-01), pages 117-144, XP009051808, ISSN: 0250-3751
- LIM ET AL: "Comparison of Polyhexamethylene Biguanide and Chlorhexidine as Monotherapy Agents in the Treatment of Acanthamoeba Keratitis", AMERICAN JOURNAL OF OPHTHALMOLOGY, ELSEVIER, AMSTERDAM, NL, vol. 145, no. 1, 8 November 2007 (2007-11-08), pages 130-135, XP022394436, ISSN: 0002-9394, DOI: 10.1016/J.AJO.2007.08.040

## Description

The present invention relates to a disinfectant composition for ophthalmic use, and in particular a composition for the daily disinfection of the ocular surface and of the ocular structures connected to it such as the conjunctival arches, lacrimal ducts, eyelid margins, eyelashes and eyebrows.

Ocular infections are quite common and can be caused by bacteria, viruses, allergies or microbiological agents. Common infections are, for example, conjunctivitis, blepharitis and trachoma. Such infections spread easily and in some cases can cause serious problems such as blurred vision, damage to the retina, damage to the cornea, damage to the optic nerve or even blindness.

Treatment of ocular infections by local antiseptics, administered topically, is known.

For example, J. Clin. Exp. Ophtalmol 2015, 6: 3 describes a study concerning the use of local antiseptics, such as polyhexamethylene biguanide and chlorhexidine digluconate, in the topical treatment of Achatamoeba keratitis. In particular, the penetration of antiseptics into rabbit corneas has been studied in artificial perfusion chambers by instillation of ocular drops of two different preparations comprising respectively 0.02% polyhexamethylene biguanide and 0.02% chlorhexidine digluconate. However, the study showed that both the examined antiseptics have very little penetration through the cornea, which could explain the fact that the treatment of Achatamoeba keratitis requires many months of topical application of such antiseptics.

Chlorhexidine is an antiseptic disinfectant with a broad spectrum of action, active towards Gram-positive and Gram-negative bacteria and also towards fungi. However, chlorhexidine is known to be an eye irritant and may cause permanent damage to the corneas. In fact, the use of chlorhexidine as a detergent during the preparation for facial surgery has caused eye pain, inflammation of the conjunctiva, loss of epithelial cells of the cornea, chronic corneal ulcers and corneal opacification.

Iodopovidone is a stable chemical complex of polyvinylpyrrolidone (povidone, PVP) and elemental iodine. Iodopovidone contains from 9.0% to 12.0% of available iodine, calculated on an anhydrous basis, and it has an antibacterial activity. Community Eye Health, Vol. 16 No. 46, 2003 describes the use of iodopovidone as an antiseptic agent for ophthalmic use. In particular, the use of a 5% iodopovidone ophthalmic solution as a preoperative topical antiseptic is described; the use of a 2.5% solution for prophylaxis against neonatal conjunctivitis; and the use of a 1.25% solution to treat certain forms of conjunctivitis.

WO02 / 087326 discloses compositions for the disinfection of contact lenses and eyewash, comprising a liquid solvent medium, a disinfectant component and a vitamin component. However, in the aforementioned application no composition comprising the disinfectants of the present invention is specifically described, nor are problems addressed related to side effects and / or to the poor disinfectant effectiveness of compositions comprising them, depending on the concentration.

It is therefore an object of the present invention to provide a disinfectant composition for the ocular surface comprising chlorhexidine or iodopovidone, free from the drawbacks of the prior art. Said object is achieved with a disinfecting composition whose main characteristics are specified in the first claim, while other characteristics are specified in the remaining claims.

An advantage of the disinfectant composition according to the present invention is that it comprises at least one disinfectant agent with a broad spectrum of action, thus effective against all the most common ocular pathogens, in a low concentration such as to avoid the damages to the conjunctiva and / or to the cornea which have been highlighted so far in cases of topical administration at a higher concentration. Moreover, thanks to the synergistic combination of the disinfectant agent with vitamin E, and / or a derivative thereof, and sodium hyaluronate, in the composition of the present invention the antiseptic efficacy is still high even at a low concentration of the disinfecting agent.

A second advantage of the composition according to this invention is that, given the low concentration of the irritant components contained in it, it is suitable for daily use both in treatment and in the prevention of ocular infections, and specifically for daily sterilisation and disinfection of the ocular surface, tear film, lacrimal ducts, conjunctival fornics, and possibly also eyelashes and eyebrows.

Further advantages and characteristics of the composition according to the present invention will be evident to the experts of the branch from the following detailed and not limiting description of its own form of realization with reference to the examples.

The disinfectant composition according to this invention is indicated for use in the treatment and/or prevention of topical ocular infections, e.g. for the daily disinfection of the ocular surface and related ocular structures as the conjunctival fornics, the lacrimal ducts, the palpebral margins, as well as eyelashes and eyebrows. The disinfectant composition according to the present invention includes a disinfectant agent, vitamin E and/or its derivative, and hyaluronic acid and/or its salt, in addition to purified water.

The disinfectant agent of the composition according to this invention is chosen in the group formed by chlorhexidine, chlorhexidine salts and iodopovidone and is present in a concentration of between 0.01% w/v and 3.0% w/v. This vitamin E and/or its derivative is present Instead, in a concentration between 0.05% w/v and 1.00% w/v; and said hyaluronic acid and/or its salt is included in a concentration between 0.01% w/v and 1.00% w/v.

In this description and in the claims, as noted, the percentage by weight/volume (% w/v) indicates the quantity expressed in grams of solute present in 100 ml of solution.

In facts it was surprisingly found that the disinfectant agents of the above specified group, if in combination with Vitamin E and/or its derivative and with Hyaluronic acid and/or its salt, are characterized by an increased ability of permeation of the cornea. In this way, the combination of the three above mentioned active ingredients increases the absorption of disinfectant agents in the tissues and therefore improves their efficacy even at a low concentration of the administered composition.

Preferably, this disinfectant agent is present in a concentration of between 0.01% w/v and 1.00% w/v. In the case of chlorhexidine, the disinfectant agent of the composition according to the present invention is advantageously present in a percentage concentration between 0.15% w/v and 0.25% w/v. In the case of iodopovidone, the disinfectant agent of the composition according to the present invention is advantageously present in a concentration between 0.40% w/v and 0.80% w/v.

The percentage concentration of such vitamin E and/or derivative is preferably between 0.10% and 0.60% w/v. The percentage concentration of such vitamin E and/or derivative may also be between 0.10% and 0.30% w/v.

According to a preferred form of invention, said Vitamin E and/or its derivative is present in the disinfectant composition of the invention at a concentration of 0.50 ± 5% w/v.

As a vitamin E derivative it can for example be used vitamin E acetate, vitamin E succinate, vitamin E TGPS, i.e. the product of the esterification of vitamin E succinate with polyethylene glycol 1000.

Hyaluronic acid and/or its salt are present in the composition according to this invention preferably in a concentration of between 0.01% and 1.00% w/v, preferably between 0.01% w/v and 0.50% w/v. Even more preferably the concentration of Hyaluronic acid and/or its salt is between 0.05% w/v and 0.30% w/v.

As hyaluronic acid salt in the composition according to this invention, sodium hyaluronate can be used.

The disinfectant composition according to the present invention is in a form suitable for topical administration, for example in the form of collyrium, eye drops, eyewash or similar.

The composition according to this invention may also include known excipients in the field of topical compositions for ophthalmic use, such as preservatives, buffer agents, antioxidant agents, viscosity regulation agents, isotonic substances.

Among preservatives, for example benzalconium chloride, benzethonium hydrochloride, chlorobutanol, EDTA, mercurial preservatives (such as thimerosal), phenyl ethyl alcohol, sodium benzoate, sodium propionate and sorbic acid can be used.

As buffer agents, the composition according to the present invention may include, for example, sodium phosphate (sodium dibasic phosphate), sodium hydrogenphosphate (dibasic sodium phosphate) and its hydrates, sodium hydroxide, sodium citrate, sodium carbonate, sodium borate, sodium dicarbonate, potassium phosphate, potassium citrate, potassium carbonate, their hydrates, boric acid, hydrochloric acid and acetic acid.

As antioxidant agents, EDTA, thiourea, sodium thiosulfate, sodium metabisulfite and sodium bisulfite can be used.

As viscosity regulators, for example, sodium carboxymethylcellulose, hydroxymethylcellulose, hydroxypropylmethylcellulose, polyethylene glycol, polyvinylpyrrolidone, polyvinyl acetic alcohol, Dextran 70, gelatine, glycerine, Polysorbate 80 can be used.

As isotonic substances, potassium chloride, propylene glycol, sodium chloride, glycerine, dextrose, Dextran 40 and Dextran 70 can be used in the disinfectant composition according to this invention.

A form of disinfectant composition for use in the treatment and/or prevention of ocular infections according to the present invention is represented by a collyrium with the following composition: Composition 1
Sodium hyaluronate 0.2% w/v
Dibasic sodium phosphate dodecahydrate 0.3% w/v
Sodium monobasic phosphate 0.066% w/v
Sodium Chloride 0.7% w/v
Vitamin E TPGS 0.5% w/v
Iodopovidone 0.6% w/v
Purified water q.s.

Another form of disinfectant composition for use in the treatment and/or prevention of ocular infections according to this invention is represented by a collyrium with the following composition: Composition 2
Sodium hyaluronate 0.2% w/v
Dibasic sodium phosphate dodecahydrate 0.3% w/v
Sodium monobasic phosphate 0.066% w/v
Sodium Chloride 0.7% w/v
Vitamin E TPGS 0.5% w/v
Chlorhexidine 0.02% w/v
Purified water q.s.

This invention will be more understood by the experts of the branch thanks to the following non-limiting examples.

### Example 1

In order to evaluate the accumulation of jodopovidone and chlorhexidine on the cornea, a modified diffusion Franz cell (Ø 9 mm, volume of receptors 5 ml, SES GmbH-Analysesysteme, Bechenheim, DE) was used. Porcine corneas were used. The eyes of pork with intact epithelium were collected at a local slaughter site, stored at 5 °c and used within 24 h post-mortem. Then, corneas with a sclera ring of about 2 mm were excised and placed on diffusion cells, with the endothelial side in the receiving compartment. This compartment consisted of isotonic phosphate buffer at pH 7.4, maintained at 37 ± 1 °C and under magnetic agitation during experiments. As a donor compartment, an aqueous iso-osmotic solution, buffered at pH 7.2, was used, of the type commonly used for fleboclysis. The concentrations of iodopovidone, hyaluronic acid, vitamin E TGPS and chlorhexidine of the tested solutions are reported in Table 1 and Table 2.

Before each trial, the corneas were balanced with the use of 0.5 mL of balanced saline solution (BSS) for irrigation for 10 min. Then, BSS was replaced by 0.5 ml of one of the A-A6 or B-B6 test solutions. Solutions A and B were used as control and applied to intact corneas. Solutions from A1 to A6 and B1 to B6 were applied to corneas with intact epithelium to evaluate the effect of the concentration on the accumulation of iodopovidone and chlorhexidine.

At predetermined intervals of 5, 10, 15, 20, 25 and 30 minutes per group the cells were dismantled, the excess donor solution removed and the corneal area available for the excluded diffusion by use of a scalpel. The corneal tissue was weighed and homogenized in 1 ml of water using 5 ml of polypropylene tubes and a Polytron PT1200E homogenizer (Kinematica AG, Lucerne, Switzerland). The tubes were then centrifuged at 12,000 rpm for 5 minutes in a refrigerated centrifuge. The liquid phase was filtered through 0.45 m nylon filters (Lida, Kenosha, USA) and analyzed by HPLC. The extraction method was validated in blank experiments with corneas with known amounts of iodopovidone and chlorhexidine. No interference peaks were detected and the recovery rate was in the range of 98-105%. All experiments were conducted by screening the diffusion cells and light samples, and were repeated at least six times.

### HPLC analysis

A new analytical method was created to analyze iodopovidone and chlorhexidine. The LC system consisted of a Jasco LC-200 equipped with a quaternary PU-2089 plus gradient pump and a standard HD-2010 plus UV detector (Jasco Europe, Cremella, LC, Italy). The selected wavelength was 264 nm. The stationary phase was a starch of Supelco C16 RP (150 mm x 4.6 mm, 5 m Supelco, Bellefonte, PA, USA) and a protection column was also used, as suggested by Vinas et al. (2004). The mobile phase was pumped with a flow of 1.2 mL / min and consisted of acetonitrile (eluent A) and 0.25 mmol KH₂PO₄ in water (eluent B).

A gradient elution was used. Initial conditions were 100% eluent A. After 1 min the mobile phase was linearly modified by adding 100% eluent B for 8 minutes and then the initial conditions were restored in the next 8 minutes.

The suitability of the analytical system was evaluated by checking the linearity range (0.70-72.4 g / ml, R² = 0.9891), the reproducibility, the detection limit (0.70 g / ml) and the quantification limit (1.2 g / ml).

### Statistical analysis

The results are expressed as mean ± SEM. The data obtained were analyzed by the Student T test for statistical significance. A p ≤0.05 value was considered significant in this study.

### Effect of VE-TPGS concentration on the corneal accumulation of iodopovidone and chlorhexidine

Solutions from A1 to A6 (Table 1) and from B1 to B6 (Table 2) were tested on corneas with integral epithelium to evaluate the correlation between the concentration of vitamin, with the same concentration of hyaluronic acid, and the corneal accumulation of iodopovidone and chlorhexidine. The results are expressed as iodopovidone nmol and chlorhexidine per mg of corneal tissue after 20 minutes of exposure. Solutions A and B were used as a control.

**Table 1-Tested solutions containing iodopovidone**

| Test solutions | Hyaluronic acid % w/v | Iodopovidone % w/v | Vit. E TPGS % w/v | Average iodopovidone accumulation mmol/mg |
|---|---|---|---|---|
| A | 0,2 | 0,6 | 0,000 | 0,2 |
| A1 | 0,2 | 0,6 | 0,010 | 0,8 |
| A2 | 0,2 | 0,6 | 0,050 | 176 |
| A3 | 0,2 | 0,6 | 0,100 | 289 |
| A4 | 0,2 | 0,6 | 0,250 | 327 |
| A5 | 0,2 | 0,6 | 0,500 | 422 |
| A6 | 0,2 | 0,6 | 1.000 | 425 |

**Table 2-Tested solutions containing chlorhexidine**

| Test solutions | Hyaluronic acid % w/v | Chlorhexidine % w/v | Vit. E TPGS % w/v | Average chlorhexidine accumulation mmol/mg |
|---|---|---|---|---|
| B | 0,2 | 0,02 | 0,000 | 0,00 |
| B1 | 0,2 | 0,02 | 0,010 | 0,03 |
| B2 | 0,2 | 0,02 | 0,050 | 0,06 |
| B3 | 0,2 | 0,02 | 0,100 | 0,09 |
| B4 | 0,2 | 0,02 | 0,250 | 0,12 |
| B5 | 0,2 | 0,02 | 0,500 | 0,24 |
| B6 | 0,2 | 0,02 | 1.000 | 0,24 |

As shown in the tables, the accumulation of both iodopovidone and chlorhexidine in the corneas, in the solutions tested according to the invention except for A1 and B1, is statistically higher than the control, and increases with an increasing concentration of VitE-TPGS up to a concentration of 0.5% w / w. No significant difference was found between A5, A6 and B5, B6.

## Claims

1. Water-based disinfectant composition for use in the treatment and / or prevention of eye infections comprising:
- at least one disinfectant selected from the group consisting of chlorhexidine, chlorhexidine salts, iodopovidone;
- vitamin E and / or derivative thereof; and
- hyaluronic acid and / or a salt thereof;
wherein said disinfectant is in a concentration of between 0.01% w/v and 3.00% w/v, said vitamin E and / or derivative thereof is in a concentration of between 0.05% and 1.00% w/v; and said hyaluronic acid and/or salt thereof is in a concentration of between 0.01% w/v and 1.00% w/v, and
wherein said derivative is selected among vitamin E acetate, vitamin E succinate, and vitamin E TPGS.

2. Water-based disinfectant composition for use in the treatment and / or prevention of eye infections according to the preceding claim, **characterized in that** said disinfectant is present in a concentration between 0.01% w / v and 1.00% w/v, said vitamin E and / or derivative thereof is present in a concentration between 0.10% and 0.60% w / v; and said hyaluronic acid and / or salt thereof is in a concentration between 0.01% w / v and 0.50% w / v.

3. Water-based disinfectant composition for use in the treatment and / or prevention of eye infections according to the preceding claim, **characterized in that** said hyaluronic acid and / or salt thereof is in a concentration of between 0.05% w / v and 0.30% w / v.

4. Water-based disinfectant composition for use in the treatment and / or prevention of eye infections according to the preceding claim, **characterized in that** said disinfectant is chlorhexidine and has a concentration between 0.15% w / v and 0.25% w /v.

5. Water-based disinfectant composition for use in the treatment and / or prevention of eye infections according to any one of claims 1 to 3, **characterized in that** said disinfectant is iodopovidone and has a concentration between 0.4% w / v and 0.8% w / v.

6. Water-based disinfectant composition for use in the treatment and / or prevention of eye infections according to any one of the preceding claims, **characterized in that** said vitamin E and / or derivative thereof is present in a concentration of 0.50 ± 5% w/v.

## Patentansprüche

1. Wasserbasierte Desinfektions-Zusammensetzung zur Anwendung bei der Behandlung und/oder Prävention von Augeninfektionen, umfassend:
- wenigstens ein Desinfiziens, ausgewählt aus der Gruppe bestehend aus Chlorhexidin, Chlorhexidinsalzen, Povidon-lod;
- Vitamin E und/oder ein Derivat davon; und
- Hyaluronsäure und/oder ein Salz davon;
wobei das besagte Desinfiziens in einer Konzentration zwischen 0,01 g/100mL und 3,00 g/100mL, das besagte Vitamin E und/oder ein Derivat davon in einer Konzentration zwischen 0,05 g/100mL und 1,00 g/100mL und die besagte Hyaluronsäure und/oder ein Salz davon in einer Konzentration zwischen 0,01 g/100mL und 1,00 g/100mL vorliegt, und
wobei das besagte Derivat ausgewählt ist aus Vitamin-E-Acetat, Vitamin-E-Succinat und Vitamin-E-TPGS.

2. Wasserbasierte Desinfektions-Zusammensetzung zur Anwendung bei der Behandlung und/oder Prävention von Augeninfektionen gemäß dem vorhergehenden Anspruch, **dadurch gekennzeichnet, dass** das genannte Desinfiziens in einer Konzentration zwischen 0,01 g/100mL und 1,00 g/100mL, das genannte Vitamin E und/oder ein Derivat davon in einer Konzentration zwischen 0,10 g/100mL und 0,60 g/100mL, und die genannte Hyaluronsäure und/oder ein Salz davon in einer Konzentration zwischen 0,01 g/100mL und 0,50 g/100mL vorhanden ist.

3. Wasserbasierte Desinfektions-Zusammensetzung zur Anwendung bei der Behandlung und/oder Prävention von Augeninfektionen gemäß dem vorhergehenden Anspruch, **dadurch gekennzeichnet, dass** die besagte Hyaluronsäure und/oder ein Salz davon in einer Konzentration zwischen 0,05 g/100mL und 0,30 g/100mL vorliegt.

4. Wasserbasierte Desinfektions-Zusammensetzung zur Anwendung bei der Behandlung und/oder Prävention von Augeninfektionen gemäß dem vorhergehenden Anspruch, **dadurch gekennzeichnet, dass** das besagte Desinfiziens Chlorhexidin ist und eine Konzentration zwischen 0,15 g/100mL und 0,25 g/100mL aufweist.

5. Wasserbasierte Desinfektions-Zusammensetzung zur Anwendung bei der Behandlung und/oder Prävention von Augeninfektionen gemäß einem der Ansprüche 1 bis 3, **dadurch gekennzeichnet, dass** das genannte Desinfiziens Povidon-lod ist und eine Konzentration zwischen 0,4 g/100mL und 0,8 g/100mL aufweist.

6. Wasserbasierte Desinfektions-Zusammensetzung zur Anwendung bei der Behandlung und/oder Prävention von Augeninfektionen gemäß einem der vorhergehenden Ansprüche, **dadurch gekennzeichnet, dass** das besagte Vitamin E und/oder ein Derivat davon in einer Konzentration von 0,50 ± 5 g/100mL vorhanden ist.

## Revendications

1. Composition désinfectante à base d'eau pour une utilisation dans le traitement et/ou la prévention d'infections oculaires, comprenant :
- au moins un désinfectant choisi dans le groupe constitué par la chlorhexidine, les sels de chlorhexidine, la povidone iodée ;
- de la vitamine E et/ou un dérivé de celle-ci ; et
- de l'acide hyaluronique et/ou un sel de celui-ci ;
dans laquelle ledit désinfectant est à une concentration comprise entre 0,01 % p/v et 3,00 % p/v, ladite vitamine E et/ou son dérivé est à une concentration comprise entre 0,05% et 1,00 % p/v ; et ledit acide hyaluronique et/ou son sel est à une concentration comprise entre 0,01 % p/v et 1,00 % p/v, et
dans laquelle ledit dérivé est choisi parmi l'acétate de vitamine E, le succinate de vitamine E, et la vitamine E TPGS.

2. Composition désinfectante à base d'eau pour une utilisation dans le traitement et/ou la prévention d'infections oculaires selon la revendication précédente, **caractérisée en ce que** ledit désinfectant est présent à une concentration comprise entre 0,01 % p/v et 1,00 % p/v, ladite vitamine E et/ou son dérivé est présente à une concentration comprise entre 0,10 % et 0,60 % p/v ; et ledit acide hyaluronique et/ou son sel est à une concentration comprise entre 0,01 % p/v et 0,50 % p/v.

3. Composition désinfectante à base d'eau pour une utilisation dans le traitement et/ou la prévention d'infections oculaires selon la revendication précédente, **caractérisée en ce que** ledit acide hyaluronique et/ou son sel est à une concentration comprise entre 0,05 % p/v et 0,30 % p/v.

4. Composition désinfectante à base d'eau pour une utilisation dans le traitement et/ou la prévention d'infections oculaires selon la revendication précédente, **caractérisée en ce que** ledit désinfectant est la chlorhexidine et a une concentration comprise entre 0,15 % p/v et 0,25 % p/v.

5. Composition désinfectante à base d'eau pour une utilisation dans le traitement et/ou la prévention d'infections oculaires selon l'une quelconque des revendications 1 à 3, **caractérisée en ce que** ledit désinfectant est la povidone iodée et a une concentration comprise entre 0,4 % p/v et 0,8 % p/v.

6. Composition désinfectante à base d'eau pour une utilisation dans le traitement et/ou la prévention d'infections oculaires selon l'une quelconque des revendications précédentes, **caractérisée en ce que** ladite vitamine E et/ou son dérivé est présent à une concentration de 0,50 ± 5 % p/v.
